# EUROPÄISCHE PATENTSCHRIFT

(11) **EP 0 979 829 B1**
(45) Veröffentlichungstag und Bekanntmachung des Hinweises auf die Patenterteilung: **16.06.2004**
(21) Anmeldenummer: 99114452.8
(22) Anmeldetag: 23.07.1999
(51) Int. Cl.: C07K 5/10, C07K 7/06, A61K 7/42

(54) **Kosmetische oder dermatologische Zubereitungen mit einem Gehalt an Oligopeptiden zur Hautaufhellung von Altersflecken bzw. zur Verhinderung der Hautbräunung, insbesondere der durch UV-Strahlung hervorgerufenen Hautbräunung**
Cosmetic or dermatologic compositions containing oligopeptides for clearing age spots or for prevention of skin tanning, in particular when caused by UV-radiations
Compositions cosmétiques ou dermatologiques contenant des oligopeptides pour effacer les marques de l'âge de la peau ou pour la prévention du bronzage provoqué par le rayonnement UV

(30) Priorität: 13.08.1998 US 132799
(43) Veröffentlichungstag der Anmeldung: 16.02.2000
(73) Patentinhaber: Beiersdorf Aktiengesellschaft, 20245 Hamburg (DE)
(72) Erfinder: Schönrock, Uwe, Dr., 23866 Nahe (DE); Max, Heiner, Dr., 22529 Hamburg (DE); Hearing, Vincent J., Dr., Clarksburg, MD-02871 (US)

(56) Entgegenhaltungen:
- WO-A-97/00892
- GB-A- 1 357 121
- US-A- 3 832 337
- KOHMURA, MASANORI ET AL: "Inhibition of angiotensin-converting enzyme by synthetic peptide fragment of human.kappa.-casein" AGRIC. BIOL. CHEM. (1990), 54(3), 835-6, 1990, XP002125436
- ZHAO, SUMIN ET AL: "A protein phosphatase-1-binding motif identified by the panning of a random peptide display library" J. BIOL. CHEM. (1997), 272(45), 28368-28372, XP002125437
- SANO M ET AL: "Identification of three extended antibody-binding segments in recombinant human muscle acetylcholine receptor alpha subunit extracellular domain 1-210" INTERNATIONAL IMMUNOLOGY,GB,OXFORD UNIVERSITY PRESS, XP002077784

## Beschreibung

Die vorliegende Erfindung betrifft die Herstellung von kosmetischen oder dermatologischen Zubereitungen mit einem Gehalt an Oligopeptiden zur Verwendung an sich bekannter Wirkstoffe, zur kosmetischen und topischen dermatologischen Hautaufhellung oder zur Verhinderung der Hautbräunung, insbesondere der durch UV-Strahlung hervorgerufenen Hautbräunung.

In einer bevorzugten Ausführungsform betrifft die vorliegende Erfindung die Herstellung von kosmetischen und dermatologischen Zubereitungen zur Prophylaxe und Behandlung kosmetischer oder dermatologischer Hautveränderungen wie z.B. der unerwünschten Pigmentierung, beispielsweise lokale Hyper- und Fehlpigmentierungen (beispielsweise Leberflecken, Sommersprossen), aber auch zur rein kosmetischen Aufhellung größerer, dem individuellen Hauttyp an sich durchaus angemessen pigmentierter Hautflächen.

Für die Pigmentierung der Haut verantwortlich sind die Melanozyten, welche in der untersten Schicht der Epidermis, dem Stratum basale, neben den Basalzellen als - je nach Hauttyp entweder vereinzelt oder aber mehr oder weniger gehäuft auftretende pigmentbildende Zellen vorzufinden sind. Melanozyten enthalten als charakteristische Zellorganellen Melanosomen, die bei Anregung durch UV-Strahlung verstärkt Melanin bilden. Dieses wird in die Keratinozyten transportiert und ruft eine mehr oder weniger ausgeprägte bräunliche oder braune Hautfarbe hervor.

Melanin wird als Endstufe eines oxidativen Prozesses gebildet, in welchem Tyrosin unter Mitwirkung des Enzymes Tyrosinase über 3,4-Dihydroxyphenylalanin (Dopa), Dopa-Chinon, Leucodopachrom, Dopachrom, 5,6-Dihydroxyindol und Indol-5,6-chinon schließlich in Melanin umgewandelt wird.

Probleme mit Hyperpigmentierung der Haut haben vielfältige Ursachen bzw. sind Begleiterscheinungen vieler biologischer Vorgänge, z.B. UV-Strahlung (z.B. Sommersprossen, *Ephelides*), genetische Disposition, Fehlpigmentierung der Haut bei der Wundheilung bzw. - vernarbung oder der Hautalterung (z.B. *Lentigines seniles*).

Es sind Wirkstoffe und Zubereitungen bekannt, welche der Hautpigmentierung entgegenwirken. Im praktischen Gebrauch sind im wesentlichen Präparate auf der Grundlage von Hydrochinon, welche aber einesteils erst nach mehrwöchiger Anwendung ihre Wirkung zeigen, deren übertrieben lange Anwendung andererseits aus toxikologischen Gründen bedenklich ist. Auch die Inhibierung der Tyrosinase mit Substanzen wie Kojisäure, Ascorbinsäure und Azelainsäure sowie deren Derivaten ist geläufig, hat aber kosmetische und dermatologische Nachteile.

Diesen Übelständen abzuhelfen, war Aufgabe der vorliegenden Erfindung.

In der Schrift WO-97/00892 wird die depigmentierende Wirkung der Signalproteine von Agutis (auch: Agoutis; Nagetierfamilie der Dasyproctidae, Vorkommen in ca. 20 Arten in den Wäldern Mittelamerikas) beschrieben.

Kohmura M. (Kohmura M. et Al., Agric. Biol. Chem. (1990), Vol. 54(3), Seiten 835-836) offenbart Peptide, die die Sequenz VVRP umfassen, und das Angiotensinkonvertierende Enzym hemmen.

GB 1357121 und US 3832337 offenbaren Peptide, die die Umwandlung von Angiotensin I in Angiotensin II hemmen und auch wertvoll als biologisch abbaubare UV-Absorba sind. Diese Peptide konnen in UV-Licht absorbierenden Präparaten zur Bräunung der Haut verwendet werden.

Als vorteilhafte Ausführungsform der vorliegenden Erfindung wird ebenfalls angesehen die Verwendung von einem oder mehreren monomeren oder homo- oder heterodimeren oder homo- oder heterotrimeren oder homo- oder heterotetrameren Oligopeptiden,
(1) wobei diesen Oligopeptiden als Monomeres eine Struktur Val-Val-Arg-Pro (bzw. VVRP) zugrundeliegt,
(2) wobei in der Sequenz Val-Val-Arg-Pro jeweils ein Valin unter Beibehaltung der Restsequenz durch eine Aminosäure aus der Gruppe Glycin, Alanin, Leucin, Isoleucin, Serin, Threonin oder Methionin, vorzugsweise Alanin, ersetzt werden kann,
(3) oder wobei in den monomeren oder homo- oder heterodimeren oder homo- oder heterotrimeren oder homo- oder heterotetrameren Oligopeptide Strukturen wie vorgenannt vorliegen mit dem Unterschiede, daß der C-Terminus und/oder der N-Terminus um bis zu 5 Aminosäuren ergänzt ist, gemäß der Struktur 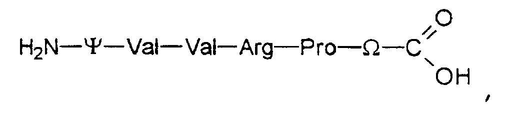 wobei Ψ und Ω unabhängig voneinander Aminosäuresequenzen von 0 bis zu 5 Aminosäuren darstellen können,
(4) oder wobei die monomeren oder homo-/hetero-di-, - tri-, oder tetrameren Oligopeptide oder Proteine am N-Terminus acyliert sind, gemäß der Struktur 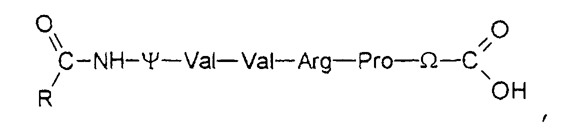 wobei Ψ und Ω unabhängig voneinander Aminosäuresequenzen von 0 bis zu 5 Aminosäuren darstellen können und R einen Verzweigten oder unverzweigten, gesättigten oder ungesättigten Kohlenwasserstoffrest, insbesondere einen Alkulrest mit 1 bis 30 Kohlenstoffatomen darstellt,
(5) oder wobei die monomeren oder homo-/hetero-di-, -tri-, oder tetrameren Oligopeptide oder Proteine am C-Terminus amidiert sind, gemäß der Struktur 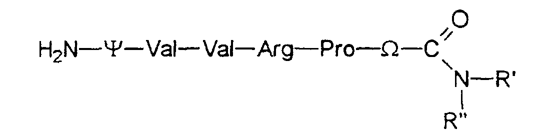 wobei Ψ und Ω unabhängig voneinander Aminosäuresequenzen von 0 bis zu 5 Aminosäuren darstellen können, und wobei R' und R" unabhängig voneinander gewählt werden können aus der Gruppe bestehend aus Wasserstoff und der verzweigten oder unverzweigten, gesättigten oder ungesättigten Kohlenwasserstoffreste, insbesondere der Alkylrest mit 1 bis 30 Kohlenstoffatomen,
(6) oder wobei die monomeren oder homo-/hetero-di-, - tri-, oder tetrameren Oligopeptide oder Proteine am C-Terminus amidiert und am N-Terminus acyliert sind, gemäß der Struktur 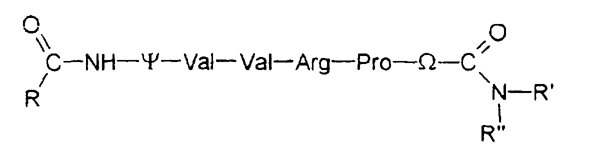 wobei Ψ und Ω unabhängig voneinander Aminosäuresequenzen von 0 bis zu 5 Aminosäuren darstellen können, R einen verzweigten oder unverzweigten, gesättigten oder ungesättigten Kohlenwasserstoffrest, insbesondere einen Alkylrest mit 1 bis 30 Kohlenstoffatomen darstellt, und wobei R' und R" unabhängig voneinander gewählt werden können aus der Gruppe bestehend aus Wasserstoff und der verzweigten oder unverzweigten, gesättigten oder ungesättigten Kohlenwasserstoffreste, insbesondere der Alkylrest mit 1 bis 30 Kohlenstoffatomen,
(7) oder wobei in monomeren oder homo-/hetero-di-, - tri-, oder tetrameren Oligopeptiden oder Proteinen Aminosäuresequenzen als einfach vorliegende oder sich mehrfach wiederholende Strukturmotive auftreten, gemäß den Strukturen 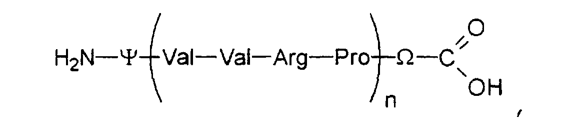 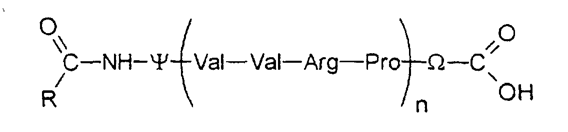 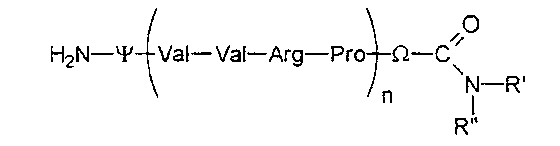 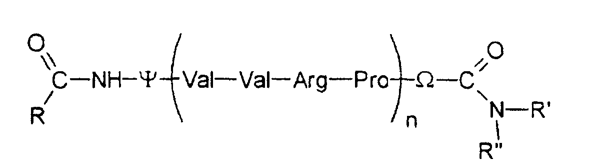 wobei Ψ eine Einfachbindung oder eine Aminosäurensequenz bis zu 5 Aminosäuren darstellen kann, Ω eine Aminosäurensequenz bis zu 15 Aminosäuren darstellen kann, n eine Zahl von 1 bis 25 darstellt, R einen verzweigten oder unverzweigten, gesättigten oder ungesättigten Kohlenwasserstoffrest, insbesondere einen Alkylrest mit 1 bis 30 Kohlenstoffatomen darstellt, und wobei R' und R" unabhängig voneinander gewählt werden können aus der Gruppe bestehend aus Wasserstoff und der verzweigten oder unverzweigten, gesättigten oder ungesättigten Kohlenwasserstoffreste, insbesondere der Alkylrest mit 1 bis 30 Kohlenstoffatomen, zur Hestellung Kosmetischer oder dermatologischer Zubereitungen gegen unerwünschte Pigmentierung der Haut.

Die die Peptidsequenz am C- bzw. N-Terminus gewünschtenfalls ergänzenden Aminosäuresequenzen konstituieren sich bevorzugt aus proteinogenen Aminosäuren.

In der Tabelle 1 werden den proteinogenen Aminosäuren ihre entsprechenden Drei- und Einbuchstabencodes gegenübergestellt.

**Tabelle**

| Drei- und Einbuchstabencodes für Aminosäuren | | |
|---|---|---|
| Aminosäure | | |
| L-Alanin | Ala | A |
| L-Valin | Val | V |
| L-Leucin | Leu | L |
| L-Isoleucin | Ile | I |
| L-Prolin | Pro | P |
| L-Tryptophan | Typ | W |
| L-Phenylalanin | Phe | F |
| L-Methionin | Met | M |
| Glycin | Gly | G |
| L-Serin | Ser | S |
| L-Tyrosin | Tyr | Y |
| L-Threonin | Thr | T |
| L-Cystein | Cys | C |
| L-Asparagin | Asn | N |
| L-Glutamin | Gln | Q |
| L-Asparaginsäure | Asp | D |
| L-Glutaminsäure | Glu | E |
| L-Lysin | Lys | K |
| L-Arginin | Arg | R |
| L-Histidin | His | H |

Dabei können das L-Selenocystein das L-Cystein und das L-Selenomethionin das L-Methionin ersetzen. Darüberhinaus können einzelne, mehrere oder auch alle Positionen auch durch die entsprechend D-konfigurierten Stereoisomere ersetzt werden.

Erfindungsgemäß besonders vorteilhaft ist beispielsweise die Verwendung von den Oligopeptiden Val-Val-Arg-Pro, Val-Val-Arg-Pro-Pro sowie Val-Val-Arg-Pro-Pro-Pro.

Erfindungsgemäß insbesondere vorteilhaft ist die Verwendung von am N-Terminus acylierten und/oder am C-Terminus amidierten Produktenden Oligopeptide Val-Val-Arg-Pro, Val-Val-Arg-Pro-Pro sowie Val-Val-Arg-Pro-Pro-Pro.

Bevorzugt sind solche acylierten Oligopeptide, die am N-Terminus mit unverzweigten Alkanoylgruppen acyliert sind.

Insbesondere bevorzugt sind 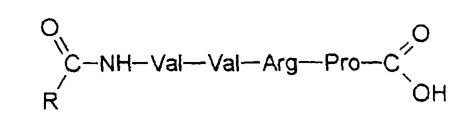 sowie 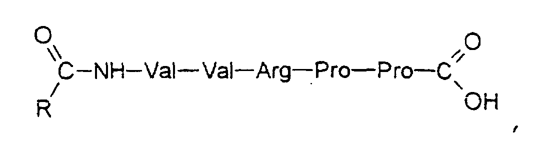 wobei R den Stearoyl oder den Palmitoylrest darstellt.

Gegebenenfalls können die erfindungsgemäß verwendbaren Peptide besser mittels DNA-Rekombinationsverfahren synthetisiert werden. Gegebenenfalls ist es jedoch besser, die erfindungsgemäß verwendbaren Peptide nach den bekannten Kettenverlängerungstechniken, wie z.B. der Festphasensynthese, z.B. an einem Merrifield-Harz o.ä., zu synthetisieren.

Um ein Peptid mittels DNA-Rekombinationstechnik zu synthetisieren, stellt man typischerweise eine DNA-Doppelstrangkette her, die für die gewünschte Aminosäuresequenz kodiert. Die Degeneration des genetischen Code gestattet die Verwendung verschiedenster Codon-Kombinationen, um die für das Produktpeptid kodierende DNA-Kette zu bilden. Einige bestimmte Codons sind für die Expression von Peptiden in bestimmten Arten von Organismen wirksamer, und man wählt die Codons vorzugsweise entsprechend derjenigen Codons, die für die Expression in der Art von Organismus, der als Wirt für den rekombinanten Vektor dienen soll, am wirksamsten sind. Es sollte jedoch jeder beliebige Satz Codons für das gewünschte Produkt kodieren, wenn auch auf etwas weniger wirksame Weise. Die Auswahl der Codons kann auch von Überlegungen bezüglich der Vektorkonstruktion abhängen, z.B. kann es nötig sein, eine bestimmte Restriktionsstelle in der DNA-Kette zu vermeiden, wenn im Anschluß an die Insertion der synthetischen DNA-Kette der Vektor mit einem Restriktionsenzym, das an solch einer Stelle schneidet, manipuliert werden soll. Auch muß man verhindern, daß Restriktionsstellen in die DNA-Kette eingebaut werden, wenn bekannt ist, daß der mit dem die DNA-Kette enthaltenden rekombinanten Vektor zu transformierende Wirtsorganismus ein Restriktionsenzym produziert, das an solch einer Stelle innerhalb der DNA-Kette schneiden würde.

Außer den Kodiersequenzen kann die hergestellte DNA-Kette weitere Sequenzen enthalten, was von Überlegungen bezüglich der Vektorkonstruktion abhängt. Typischerweise wird eine DNA-Kette mit Linkern an den Enden hergestellt, um das Insertieren in Restriktionsstellen innerhalb eines Klonierungsvektors zu erleichtern. Die DNA-Kette kann so aufgebaut sein, daß die gewünschte Sequenz als Teil eines Fusionspeptids kodiert wird, und kann, falls dies der Fall ist, endständige Sequenzen enthalten, die für Aminosäurerest-Sequenzen kodieren, die als Stellen für das proteolytische Processing dienen, wodurch das gewünschte Peptid proteolytisch vom Rest des Fusionspeptids abgespalten werden kann. Die endständigen Teile der synthetischen DNA-Kette können auch geeignete Start- und Stop-Signale enthalten.

Um die gewünschte DNA-Kette aufzubauen, werden Oligonucleotide nach traditionellen Methoden, wie den bei T. Maniatis et al. COLD SPRING HARBOR LABORATORY MANUAL, Cold Spring Harbor, N.Y. (1982) (im folgenden Text mit CSH bezeichnet) beschriebenen Verfahren konstruiert. Sense- und Antisense-Oligonucleotidketten mit einer Länge von bis zu 70 Nucleotidresten werden vorzugsweise mit automatischen Synthesizern, wie dem DNA-Synthesizermodell 380B von Applied Biosystems synthetisiert. Der Aufbau der Oligonucleotidketten ist derart, daß die Sense- und Antisense-Oligonucleotide miteinander über Wasserstoffbrückenbindung zwischen komplementären Basenpaaren assoziieren und so doppelsträngige Ketten bilden. Diese Oligonucleotide werden dann mit dem Vektor ligiert.

Der zwecks Einbau der DNA-Kette zu rekombinierende Klonierungsvector wird je nach seiner Lebensfähigkeit und Expression in einem Wirtsorganismus oder einer Wirtszellinie ausgewählt, und die Art, auf die die DNA-Kette insertiert wird, hängt von wirtsspezifischen Faktoren ab. Soll zum Beispiel die DNA-Kette in einen Vektor zweck Insertion in eine Prokaryontenzelle, wie z.B. E. coli, insertiert werden, so wird die DNA-Kette in 3'-Richtung einer Promotersequenz, einer Shine-Delgarno-Sequenz (oder Ribosomen-Bindungsstelle) innerhalb eines 5'-nichttranslatierten Teils und eines ATG-Startcodons insertiert werden. Das ATG-Startcodon ist in einem entsprechenden Abstand von der Shine-Delgarno-Sequenz, und die Kodiersequenz wird leserastergerecht mit dem ATG-Startcodon angeordnet. Der Klonierungsvektor stellt auch eine 3'nichttranslatierte Region und eine Transkriptionsterminationsstelle bereit. Die Translationsterminationsstelle könnte entweder von der synthetischen DNA oder von dem Klonierungsvektor bereitgestellt werden. Zwecks Insertion in eine Eukaryontenzelle, wie z.B. eine Hefezelle oder eine von einem höheren Lebewesen erhaltene Zellinie, wird die kodierende Oligonucleotidsequenz in einem geeigneten Abstand von einer Capping-Stelle und leserastergerecht mit einem ATG-Startsignal angeordnet. Der Klonierungsvektor stellt auch eine 3'-nichttranslatierte Region und eine Polyadenylierungsstelle bereit. Eine Translationsterminationsstelle könnte entweder von der synthetischen DNA oder von dem Vektor bereitgestellt werden.

Abkömmlinge von Prokaryontenvektoren, wie pBR322, pMB9, Col El, pCRI, RP4 sowie der Phage lambda, sind verfügbar, um eine DNA-Kette, die lange genug ist, um für interessierende Peptide zu kodieren, so zu insertieren, daß zumindest eine gewisse Expression des kodierten Peptids im wesentlichen gewährleistet ist. Solche Vektoren werden typischerweise so konstruiert oder modifiziert, daß sie über eine einzelne Restriktionsstelle, bzw. einzelne Restriktionsstellen, in einer geeigneten Position bezüglich des Promoters, z.B. des Lac-Promoters, verfügen. Die DNA-Kette kann in solch eine Restriktionsstelle mittels geeigneter Linker insertiert werden, wobei die Produktion von Peptid in einer mit dem rekombinanten Vektor transformierten Prokaryontenzellinie im wesentlichen garantiert ist. Für ein korrektes Leseraster können die Enden der für das Peptid kodierenden Sequenz mit Linkern verschiedener Länge versehen sein. Kassetten, die Sequenzen wie z.B. die 5'-Region des Lac Z Gens (inklusive Operator, Promoter, Transkriptionsstartstelle, Shine-Delgarno-Sequenz und Translationsinitiationssignal), die Regulatorregion des Tryptophan-Gens (trp-Operator, Promoter, Ribosomenbindungsstelle und Translationsinitiator) sowie ein diese beiden Promoter enthaltendes Fusionsgen, das mit trp-Lac oder üblicherweise Tac-Promoter bezeichnet wird, enthalten, und in die eine synthetische DNA-Kette bequem insertiert werden kann, sind verfügbar.

Auf ähnliche Weise existieren Vektoren für die Transformation von Eukaryonten, z.B. das clonierte Rinderpapillomavirus-Genom, die clonierten Genome von Mäuse-Retroviren, sowie Kassetten für Eukaryonten, wie das pSV-2 gpt System (beschrieben von Mulligan und Berg, NATURE, Band 277, Seiten 108-114, 1979), das Klonierungssystem nach Okayama-Berg (MOL. CELL. BIOL., Band 2, Seiten 161-170, 1982) und der in jüngster Zeit vom Genetics Institute beschriebene Expressionsklonierungsvektor (SCIENCE, Band 228, Seiten 810-815, 1985). Durch diese wird zumindest eine gewisse Expression des Peptids in der transformierten Eukaryontenzellinie im wesentlichen gewährleistet.

Die gewünschten Peptide können jedoch auch so hergestellt werden, daß das Peptid anfänglich als Abschnitt eines von einem Gen kodierten Fusionspeptid produziert wird. In solch einem Fall wird die DNA-Kette so konstruiert, daß das exprimierte Peptid über die Peptidsequenz flankierende Stellen für das enzymatische Processing, oder, was häufiger ist, über eine Processing-Stelle an einer Seite des gewünschten Peptids verfügt. Eine für ein Peptid kodierende DNA-Kette kann zum Beispiel in das Beta-Galactosidase-Gen zwecks Insertion in E. coli insertiert werden, und in diesem Fall wird das exprimierte Fusionspeptid dann mit geeigneten proteolytischen Enzymen gespalten, um das Peptid aus Beta-Galactosidase-Peptid-Sequenzen freizusetzen.

Die Peptide können jedoch auch durch geeignete Kettenverlängerungs- oder Kopplungsverfahren hergestellt werden, zum Beispiel durch reine Festphasentechniken, teilweise Festphasentechniken, Fragmentkondensation oder klassische Lösungskopplungen. Die Techniken der reinen Festphasensynthese werden in dem Lehrbuch "Solid-Phase Peptide Synthesis" Stewart & Young, Pierce Chemical Company, Rockford, Ill., 1984 erläutert und durch die Offenbarung von US-Patent Nr. 4,105,603, ausgegeben am 8. August 1978, veranschaulicht. Das Syntheseverfahren der Fragmentkondensation wird in US-Patent Nr. 3,972,859, ausgegeben am 3. August 1976, veranschaulicht. Weitere zur Verfügung stehende Synthesen werden durch die US-Patente Nr. 3,842,067, ausgegeben am 15. Oktober 1974, sowie 3,862,925, ausgegeben am 28. Januar 1975, veranschaulicht.

Den Synthesen des Kopplungstyps gemeinsam ist, daß die labilen Seitenkettengruppen der verschiedenen Aminosäurereste mit geeigneten Schutzgruppen geschützt werden, die verhindern, daß an dieser Stelle eine chemische Reaktion stattfindet, bis die Gruppe schließlich entfernt wird. Auch ist ihnen üblicherweise gemeinsam, daß eine Alpha-Aminogruppe durch eine aminoterminale Blockierungsgruppe an einer Aminosäure oder einem Fragment geschützt wird, während diese(s) an der Carboxylgruppe reagiert, worauf man die aminoterminale Blockierungsgruppe selektiv entfernt, wonach an dieser Stelle eine Reaktion stattfinden kann. Dementsprechend wird häufig als Syntheseschritt eine Zwischenverbindung produziert, die jeden dieser Aminosäurereste in dessen gewünschter Sequenz in der Peptidkette enthält, wobei die Schutzgruppen für die Seitenkette oder die terminalen Blockierungsgruppen mit den jeweiligen Resten verbunden sind.

Bei der Wahl einer bestimmten, bei der Synthese der Peptide zur verwendenden Schutzgruppe für die Seitenkette hält man sich an folgende Regeln: (a) die Schutzgruppe soll gegenüber dem Reaktionsmittel und unter den für die Entfernung der Alpha-Amino-Schutzgruppe während jedes Syntheseschritts gewählten Reaktionsbedingungen stabil sein, (b) die Schutzgruppe soll ihre schützenden Eigenschaften beibehalten und unter Kopplungsbedingungen nicht abgespalten werden, sowie (c) die Schutzgruppe für die Seitenkette soll nach der Synthese mit der gewünschten Aminosäuresequenz unter Reaktionsbedingungen, die die Peptidkette nicht verändern, entfernbar sein.

Die Peptide werden vorzugsweise mittels Festphasensynthese, wie z.B. der bei Merrifield, J. AM. CHEM. SOC., Band 85, Seiten 2149 (1963), beschriebenen Festphasensynthese hergestellt, obwohl wie bereits bemerkt auch andere entsprechende fachbekannte chemischen Synthesen verwendet werden können. Die Festphasensynthese beginnt am C-terminalen Ende des Peptids durch Ankoppeln einer geschützten Alpha-Aminosäure an ein geeignetes Harz. Solch ein Ausgangsmaterial kann dadurch hergestellt werden, daß man ein Alpha-Amino-geschütztes Val mittels Esterbindung an ein chlormethyliertes Harz oder Hydroxymethylharz oder durch eine Amidbindung an ein Butylhydroxyanisol(BHA)-Harz oder MBHA-Harz anheftet. Die Herstellung des Hydroxymethylharzes wird bei Bodansky et al., CHEM. IND. (London) Band 38, S. 1597-98 (1966) beschrieben. Chlormethylierte Harze sind im Handel von Bio Rad Laboratories, Richmond, Kalifornien und von Lab. Systems, Inc. erhältlich. Die Herstellung solch eines Harzes wird bei Stewart et al., "Solid Phase Peptide Synthesis" (Freeman & Co., San Francisco 1969), Kapitel 1, Seiten 1-6, beschrieben. BHA- und MBHA-Harzträger sind im Handel erhältlich und werden im allgemeinen nur dann verwendet, wenn das gewünschte Polypeptid, das hergestellt wird, über ein Alpha-Carboxamid am C-terminalen Ende verfügt.

Bei der Festphasensynthese der Peptide verwendete Aktivierungsmittel sind auf dem Gebiet der Peptidsynthese gut bekannt. Geeignete Aktivierungsmittel sind zum Beispiel (1) Carbodiimide, wie N,N-Diisopropylcarbodiimid, N,N-Dicyclohexylcarbodiimid (DCCI); (2) Cyanamide, wie N,N-Dibenzylcyanamid; (3) Ketimine; (4) Isoxazoliumsalze, wie N-Ethyl-5-phenylisooxazolium-3-sulfonat; (5) einkernige stickstoffhaltige heterocyclische Amide mit aromatischem Charakter, die im Ring ein bis vier Stickstoffe enthalten, wie Imidazolide, Pyrazolide und 1,2,4-Triazolide. Zu brauchbaren heterocyclischen Amiden gehören im einzelnen N,N-Carbonyldiimidazol, N,N-Carbonyldi-1,2,4-triazol; (6) alkoxyliertes Acetylen, wie Ethoxyacetylen; (7) Reagenzien, die mit dem Carboxylteil der Aminosäure ein Mischanhydrid bilden, wie Chlorameisensäureethylester und Chlorameisensäureisobutylester, sowie (8) Reagenzien, die mit dem Carboxylteil der Aminosäure einen aktivierten Ester bilden, wie stickstoffhaltige Heterocyclen mit einer Hydroxylgruppe an einem Ringstickstoff, z.B. N-Hydroxyphthalimid, N-Hydroxysuccinimid und 1-Hydroxybenzotriazol (HOBT). Weitere Aktivierungsmittel und ihre Verwendung bei der Peptidkopplung sind bei Schroder & Lubke, oben, in Kapitel III und bei Kapoor, J. PHAR. SCI., Band 59, Seiten 1-27 (1970) beschrieben.

Jede geschützte Aminosäure bzw. Aminosäuresequenz wird in dem Festphasenreaktor in einem ungefähr zweifachen oder größeren Überschuß vorgelegt, und bei dem Medium, in dem gekoppelt wird, kann es sich um Dimethylformamid (DMF)/CH₂Cl₂ (1:1) oder um DMF oder CH₂Cl₂ allein handeln. Bei unvollständigem Koppeln wird der Kopplungsvorgang vor der Entfernung der Alpha-Amino-Schutzgruppe wiederholt, bevor die nächste Aminosäure angekoppelt wird. Bei händischer Kopplung wird der Erfolg jeder Stufe mittels Ninhydrin-Reaktion nach E. Kaiser et al., ANAL. BIOCHEM., Bd. 34, S. 595 (1970) kontrolliert.

Nach Vervollständigung der gewünschten Aminosäuresequenz wird die Peptid-Zwischenstufe von dem Harzträger durch Versetzen mit einem Reagens wie flüssigem Fluorwasserstoff entfernt, wodurch nicht nur das Peptid vom Harz abgespalten wird, sondern auch alle restlichen Seitenkettenschutzgruppen X², X³, X⁴, X⁵, X⁶, X⁷, X⁸ und X⁹ sowie die Alpha-Amino-Schutzgruppe X¹ abgespalten werden, wodurch man das Peptid erhält.

Es ist jedoch auch möglich, die Peptid-Zwischenstufe von dem Harzträger alkoholytisch abzutrennen, wonach der erhaltene C-terminale Alkylester mittels Hydrolyse in die Säure umgewandelt wird. Jegliche Seitenkettenschutzgruppe kann dann wie oben beschrieben oder nach anderen Verfahren, zum Beispiel katalytische Reduktion (z.B. Pd auf BaSO₄), abgespalten werden. Wird mit Fluorwasserstoff abgespalten, so enthält das Reaktionsgefäß Anisol und Methylethylsulfid zum Abfangen.

Einzelpeptide können beispielsweise vorteilhaft mit Hilfe eines automatischen MilliGen 9050 Peptidsynthesizers, überlappende Peptide mit Hilfe eines Multiplen Peptidsynthesizers SMPS 350 (ZINSSER Analytik) durch Festphasensynthese (R.B.Merrifield (1966) J.Am.Chem.Soc. 85, 2149) nach der Fmoc/But-Strategie (E. Atherton, R.C.Sheppard; "Solid Phase Peptide Synthesis: A Practical Approach", IRL Press, Oxford, England (1989) hergestellt werden.

Kupplungs-und Abspaltungsschritte können dabei mittels eines on-line UV-monitoring kontrolliert werden. Als Festphasenmatrix für freie Peptide kann beispielsweise vorteilhaft ein p-Alkoxybenzylalkohol-modifiziertes Polystyrolharz (S.-S. Wang; J. Am. Chem. Soc. 95 (1973), S.1328), für Peptidamide das Rink-Amid-Harz (H.Rink; Tetrahedron Lett. 28 (1987), S.3787) verwendet werden. Als Kupplungsreagens kann vorteilhaft TBTU (2-(1H-Benzotriatol-1-yl)-1,1,3,3-tetramethyluroniumtetrafluoroborat) (R.Knorr, A.Trzeciak, W.Bannwarth, D.Gillesen; Tetrahedron Lett. 30 (1989), S. 1927) eingesetzt werden. Übliche Kupplungszeiten betragen jeweils 10 - 30, insbesondere ca. 20 Minuten. Nach Ende der Synthese können die Peptide beispielsweise mit Hilfe von Trifluoressigsäure und gewünschtenfalls Zusatzsubstanzen (z.B. Phenol, Thioanisol, aber auch Wasser) vom Harz abgespalten sowie gleichzeitig von allen Schutzgruppen befreit.

Die Reaktionslösung kann, z.B. durch Eintropfen in eiskalten Diethylether ein Rohprodukt ergeben, welches, gewünschtenfalls unter Waschen mit einer geeigneten Waschflüssigkeit, z.B. kaltem Diethylether, gereinigt und hernach lyophilisiert werden kann. Das rohe Peptid wird dann vorteilhaft durch präparative Reversed-Phase-Hochleistungsflüssigkeit-schromatographie (RP-HPLC) an einer C18-Säule (250 x 10 mm) über ein Acetonitril/0,05% Trifluoressigsäure/Wasser-System zu einem Reinheitsgrad von > 98% hochgereinigt.

Durch Umsetzung der N-Terminal entschützten harzgebundenen Peptide mit Säurechloriden oder Säureanhydriden (z.B. Palmitoylchlorid bzw. Laurylchlorid) in einem geeigneten Lösungsmittel (z.B. Dimethylformamid/N-Methylmorpholin) können am N-Terminus acylierte Peptide in hohen Ausbeuten erhalten werden.

Die erfindungsgemäß verwendeten, kosmetisch oder pharmazeutisch unbedenklichen Oligopeptide, hernach, ungeachtet, ob eine Einzelsubstanz oder ein Isomerengemisch oder ein Gemisch verschiedener Einzelsubstanzen vorliegt, kollektiv auch "erfindungsgemäß verwendeter Wirkstoff" genannt, haben sich als hervorragende Wirkstoffe gegen unerwünschte Pigmentierung, insbesondere lokale Hyperpigmentierung, und zwar sowohl präventiv wie auch im Sinne einer Behandlung, erwiesen.

Erfindungsgemäß kann der Gehalt an erfindungsgemäß verwendetem Wirkstoff in den kosmetischen oder topischen dermatologisochen Zubereitungen 0,000001 - 10 Gew.-%, bevorzugt 0,0001 - 1 Gew.-%, insbesondere 0,0001 - 0,1 Gew.-% betragen, bezogen auf das Gesamtgewicht der Zubereitungen.

Es hat sich überraschenderweise herausgestellt, daß der erfindungsgemäß verwendete Wirkstoff die der Erfindung zugrundeliegenden Aufgaben erfüllt. Bei Anwendung des erfindungsgemäß verwendeten Wirkstoffes bzw. kosmetischer oder topischer dermatologischer Zubereitungen mit einem wirksamen Gehalt an erfindungsgemäß verwendetem Wirkstoff ist eine wirksame Prophylaxe gegen unerwünschte Pigmentierung möglich. Es ist erfindungsgemäß vorteilhaft, den erfindungsgemäß verwendeten Wirkstoff zur herstellung von kosmetischen oder dermatologischen Zubereitungen gegen unerwünschter Hautpigmentierung, also beispielsweise *Lentigines seniles*, zu verwenden.

Die Prophylaxe bzw. die kosmetische oder dermatologische Behandlung mit dem erfindungsgemäß verwendeten Wirkstoff bzw. mit den kosmetischen oder topischen dermatologischen Zubereitungen mit einem wirksamen Gehalt an erfindungsgemäß verwendetem Wirkstoff erfolgt in der üblichen Weise, und zwar dergestalt, daß der erfindungsgemäß verwendete Wirkstoff bzw. die kosmetischen oder topischen dermatologischen Zubereitungen mit einem wirksamen Gehalt an erfindungsgemäß verwendetem Wirkstoff auf die betroffenen Hautstellen aufgetragen wird.

Vorteilhaft kann der erfindungsgemäß verwendete Wirkstoff eingearbeitet werden in übliche kosmetische und dermatologische Zubereitungen, welche in verschiedenen Formen vorliegen können. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W) oder Öl-in-Wasser-in-Öl (O/W/O), eine Hydrodispersion oder Lipodispersion, ein Gel, einen festen Stift oder auch ein Aerosol darstellen.

Erfindungsgemäß hergestellte Emulsionen z.B. in Form einer Crème, einer Lotion, einer kosmetischen Milch sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Es ist auch möglich und vorteilhaft den erfindungsgemäß verwendeten Wirkstoff in wäßrige Systeme bzw. Tensidzubereitungen zur Reinigung der Haut und der Haare einzufügen.

Es ist dem Fachmanne natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Medizinische topische Zusammensetzungen hergestellt im Sinne der vorliegenden Erfindung enthalten in der Regel ein oder mehrere Medikamente in wirksamer Konzentration. Der Einfachneit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Es ist dabei ebenfalls von Vorteil, den erfindungsgemäß verwendeten Wirkstoff als Zusatzstoff zu Zubereitungen zu geben.

Entsprechend können kosmetische oder topische dermatologische Zusammensetzungen hergestellt im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäß hergestellten Zusammensetzungen als Grundlage für pharmazeutische Formulierungen zu verwenden.

Günstig sind gegebenenfalls auch solche kosmetischen und dermatologischen Zubereitungen, die in der Form eines Sonnenschutzmittels vorliegen. Vorzugsweise enthalten diese neben dem erfindungsgemäß verwendeten Wirkstoff zusätzlich mindestens eine UVA-Filtersubstanz und/oder mindestens eine UVB-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden beispielsweise in Tagescrèmes gewöhnlich UV-A- bzw. UV-B-Filtersubstanzen eingearbeitet.

Vorteilhaft können erfindungsgemäß hergestellte Zubereitungen Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen.

Die UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher,
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi (2-ethylhexyl) ester;
- 2,4,6-Trianilino-(p-carbo-2'-ethyl-1'-hexyloxy)-1,3,5-triazin

Als wasserlösliche Substanzen sind vorteilhaft:
- 2-Phenylbenzimidazol-5-sulfonsäure und deren Salze, z.B. Natrium-, Kalium- oder Triethanolammonium-Salze,
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die erfindungsgemäß Verwendung finden können, soll selbstverständlich nicht limitierend sein.

Gegenstand der Erfindung ist auch die Herstellung von einer Kombination eines UVA-Filters wie im Anspruch 1 gezeigt mit einem UVB-Filter bzw. eine erfindungsgemäßes kosmetische oder dermatologische Zubereitung, welche auch einen UVB-Filter enthält.

Es kann auch von Vorteil sein, in erfindungsgemäß hergestellten Zubereitungen UVA-Filter einzusetzen, die üblicherweise in kosmetischen und/oder dermatologischen Zubereitungen enthalten sind. Bei solchen Filtersubstanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Kosmetische und/oder dermatologische Zubereitungen hergestellt im Sinne der vorliegenden Erfindung können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Die erfindungsgemäß hergestellten kosmetischen und dermatologischen Zubereitungen können kosmetische Wirk-, Hilfs- und/oder Zusatzstoffe enthalten, wie sie üblicherweise in solchen Zubereitungen verwendet werden, z.B. Antioxidationsmittel, Konservierungsmittel, Bakterizide, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Pelyrole, Polymere, Schaumstabilisatoren, Elektrolyte, organische Lösungsmittel oder Silikonderivate.

Es ist ebenfalls vorteilhaft, den Zubereitungen hergestellt im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Alanindiessigsäure, Flavonoide, Polyphenole, Catechine, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoëharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern die kosmetische oder dermatologische Zubereitung hergestellt im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder - monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkybenzoat und 2-Ethylhexylisostearat, Mischungen aus C₁₂₋₁₅-Alkybenzoat und Isotridecylisononanoat sowie Mischungen aus C₁₂₋₁₅-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäß hergestellten Zubereitungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäß verwendete Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrigalkoholischen oder alkoholischen Gelen vorzugweise ein Polyacrylat ist.

Feste Stifte enthalten z.B. natürliche oder synthetische Wachse, Fettalkohole oder Fettsäureester.

Übliche Grundstoffe, welche für die Verwendung als kosmetische Stifte im Sinne der vorliegenden Erfindung geeignet sind, sind flüssige Öle (z.B. Paraffinöle, Ricinusöl, Isopropylmyristat), halbfeste Bestandteile (z.B. Vaseline, Lanolin), feste Bestandteile (z.B. Bienenwachs, Ceresin und Mikrokristalline Wachse bzw. Ozokerit) sowie hochschmelzende Wachse (z.B. Carnaubawachs, Candelillawachs)

Als Treibmittel für aus Aerosolbehältern versprühbare kosmetische und/oder dermatologische Zubereitungen hergestellt im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Kosmetische Zubereitungen hergestellt im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickung-smittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder - distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Die nachfolgenden Beispiele sollen die vorliegende Erfindung verdeutlichen.

### Herstellungsbeispiel 1

### Synthese in einer Schüttelente (geeignet für größere Syntheseansätze)

0,5 g Rink-Amid-Harz (0.2 mMol/g) werden in einer Schüttelente mit DMF gut gewaschen, danach mit 5 ml 20% (v/v) Piperidin/ DMF-Lösung versetzt. Nach 3 Minuten wird abgesaugt und nochmals für 5 Min mit 5 ml 20% Piperidin/DMF versetzt. Nach intensivem Waschen mit DMF (ca. 50 ml) wird das Harz mit 108,3 mg Fmoc-Pro-OH und 128,1 mg TBTU in 2 ml DMF versetzt. Nach Zugabe von 1,25 ml 0,7 M N-Methylmorpholin-Lösung wird für 30 Minuten geschüttelt. Nach Waschen des Harzes wird die Vollständigkeit der Reaktion durch einen Kaiser-Test überprüft.

Nach Abspalten der Fmoc-Schutzgruppe mit 20% Piperidin/DMF werden nachfolgend 259.5 mg Fmoc-Arg(Pbf)-OH (0.4 mMol), 135.8 mg (0,4 mMol) Fmoc-Val-OH und weitere 135.8 mg (0.4 mMol) Fmoc-Val-OH nach oben beschriebenen Schema gekuppelt. Die Kupplungseffizienz wird dabei jedes Mal mit Hilfe des Kaiser-Tests überprüft. Nach Deblockierung der Fmoc-Schutzgruppe wird das Harz gut mit DMF gewaschen und schliesslich getrocknet. Die Abspaltung der Schutzgruppe und des Peptids vom Harz wird durch 2 ml Trifluoressigsäure/Thioanisol/Phenol/Wasser 90:5:3:2 (vlv) vorgenommen. Nach 2 stündiger Reaktionszeit wird filtriert und das Filtrat direkt in 30 ml eisgekühlten Diethylether eingetropft. Nach Zentrifugation wird der Niederschlag gut mit Diethylether gewaschen und wiederum zentrifugiert. Der Vorgang wird dreimal wiederholt. Nach Lösen des Pellets in tert.-Butanol/Wasser (4:1, v/v) wird lyophilisert.

Das farblose Lyophilsat wird in 5 ml deionisiertem Wasser aufgenommen und nochmals gefriergetrocknet.

### Herstellungsbeispiel 2

### Synthese von H-VVRP-NH₂ (Synthese mit Synthesizer)

Eine Hochdruckglassäule des MilliGen 9050 Peptidsynthesizers wird mit 0,5g Rink-Amid-Harz (Beladung 0.2 mMol/g), dem 2,5 g glassbeads (0,150 - 212 µm, Fa. Sigma), beigemischt wurden, befüllt. Durch Zugabe von 14,8 ml 20% (v/v) Piperidin/ Dimethylformamid (Flußrate 7,4ml/min) wird nun die N-terminale Fmoc-Schutzgruppe entfernt. Nach Waschen mit Dimethylformamid (5 min a 7.4 ml/min) werden 108,3 mg (0,4 mMol) Fmoc-Prolin und 128 mg TBTU (0,4 mMol) in 1,25ml 0,7 M N-Methylmorpholinlösung in DMF gelöst. Nach 3 min wird die so voraktivierte Lösung im Recycling-Verfahren auf die Säule gepumpt und für 20 min gekuppelt. Nach Waschen mit DMF wird durch Zugabe von 20%Piperidin in DMF wiederum die N^{α}-Fmoc-Gruppe abgespalten. Als nächstes Aminosäurederivat werden 259,5 mg Fmoc-Arg(Pbf)-OH (0,4 mMol) wie obenstehend gekuppelt. Nach Kupplung Von Fmoc-Val-OH und einem weiteren Fmoc-Val-OH (je 135,8 mg, 0,4 mMol) wird wiederum die Fmoc-Gruppe abgespalten, das Harz gut mit DMF gewaschen, aus der Säule entnommen und gut getrocknet. Die Abspaltung der Schutzgruppe und des Peptids vom Harz wird durch 2 ml Trifluoressigsäure/Thioanisol/Phenol/Wasser 90:5:3:2 (v/v) vorgenommen. Nach 2 stündiger Reaktionszeit wird filtriert und das Filtrat direkt in 30 ml eisgekühlten Diethylether eingetropft. Nach Zentrifugation wird der Niederschlag gut mit Diethylether gewaschen und wiederum zentrifugiert. Der Vorgang wird dreimal wiederholt. Nach Lösen des Pellets in tert.-Butanol/Wlasser (4:1, v/v) wird lyophilisiert. Das farblose Lyophilisat wird nochmals in 5 ml deonisiertem Wasser aufgenommen und nochmals gefriergetrocknet.

Abkürzungen:
- Fmoc:: 9-Fluorenylmethoxycarbonyl-Rest
- Rink-Amid-Harz:: 4-(2',4'-Dimethylphenyl-Fmoc-aminomethyl)-phenoxy-Harz
- Fmoc-Arg(Pbf)-OH:: N^{α}-Fmoc-N^{G}-2,2,4,6,7-pentamethyldihydrobenzofuran-5-sulfonyl-Arginin
- TBTU:: 2-(1H-Benzotriatol-1-yl)-1,1,3,3-tetramethyluroniumtetrafluoroborat
- DMF: Dimethylformamid

### Beispiel 1

| W/O Creme | |
|---|---|
| | Gew.% |
| Paraffinöl (DAB 9) | 10,00 |
| Petrolatum | 4,00 |
| Wollwachsalkohol | 1,00 |
| PEG-7-Hydriertes Rizinusöl | 3,00 |
| Aluminiumstearat | 0,40 |
| H-VVRP-NH₂ | 0,01 |
| Glycerin | 2,00 |
| Konservierungsmittel, Farbstoffe, | q.s. |
| Parfüm | |
| Wasser | ad 100,00 |

### Beispiel 2

| W/O Lotion | |
|---|---|
| | Gew.-% |
| Paraffinöl (DAB 9) | 20,00 |
| Petrolatum | 4,00 |
| Glucosesesquiisostearat | 2,00 |
| Aluminiumstearat | 0,40 |
| H-VVRP-NH₂ | 0,005 |
| α-Tocopherylacetat | 1,00 |
| Glycerin | 5,00 |
| Konservierungsmittel, Farbstoffe, Parfüm | q.s. |
| Wasser | ad 100,00 |

### Beispiel 3

| O/W Lotion | |
|---|---|
| | Gew.-% |
| Paraffinöl (DAB 9) | 8,00 |
| Isopropylpalmitat | 3,00 |
| Petrolatum | 4,00, |
| Cetylstearylalkohol | 2,00 |
| PEG 40 Rizinusöl | 0,50 |
| Natriumcetylstearylsulfat | 0,50 |
| Natrium Carbomer | 0,40 |
| H-VVRP-NH₂ | 0,001 |
| Glycerin | 3,00 |
| α-Tocopherol | 0,20 |
| Octylmethoxycinnamat | 5,00 |
| Butylmethoxydibenzoylmethan | 1,00 |
| Konservierungsmittel, Farbstoffe, Parfüm | q.s. |
| Wasser | ad 100,00 |

### Beispiel 4

| O/W Creme | |
|---|---|
| | Gew.-% |
| Paraffinöl (DAB 9) | 7,00 |
| Avocadoöl | 4,00 |
| Glycerylmonostearat | 2,00 |
| Acetyl-VVRP-NH₂ | 0,01 |
| Titandioxid | 1,00 |
| Natriumlactat | 3,00 |
| Glycerin | 3,00 |
| Konservierungsmittel, Farbstoffe, Parfüm | q.s. |
| Wasser | ad 100,00 |

### Beispiel 5

| Liposomenhaltiges Gel | |
|---|---|
| | Gew.-% |
| Lecithin | 6,00 |
| Schibutter | 3,00 |
| H-VVRP-NH₂ | 0,005 |
| α-Tocopherol | 0,20 |
| Biotin | 0,08 |
| Natriumcitrat | 0,50 |
| Glycin | 0,20 |
| Harnstoff | 0,20 |
| Natrium PCA | 0,50 |
| Hydrolysiertes Kollagen | 2,00 |
| Xanthan Gummi | 1,40 |
| Sorbitol | 3,00 |
| Konservierungsmittel, Farbstoffe, Parfüm | q.s. |
| Wasser | ad 100,00 |

### Beispiel 6

| Gel | |
|---|---|
| | Gew.-% |
| Carbopol 934 P | 2,00 |
| Triethanolamin | 3,00 |
| Acetyl-VVRP-NH₂ | 0,001 |
| α-Tocopherylacetat | 0,20 |
| Polyoxyethylensorbitanfettsäureester (Tween 20) | 0,50 |
| Glycerin | 2,00 |
| Natrium PCA | 0,50 |
| Hydrolysiertes Kollagen | 2,00 |
| Konservierungsmittel, Farbstoffe, Parfüm | q.s. |
| Wasser | ad 100,00 |

### Beispiel 7

| Sonnenschutzemulsion | |
|---|---|
| | Gew.-% |
| Cyclomethicon | 2,00 |
| Cetyldimethicon Copolyol | 0,20 |
| PEG 22-Dodecyl Copolymer | 3,00 |
| Paraffinöl (DAB 9) | 2,00 |
| Caprylsäure-/Caprinsäure | 5,80 |
| Triglycerid | |
| Octylmethoxycinnamat | 5,80 |
| Butyl-methoxy-dibenzoylmethan | 4,00 |
| Acetyl-VVRP | 0,001 |
| α-Tocopherylacetat | 0,50 |
| ZnSO₄ | 0,70 |
| Na₄EDTA | 0,30 |
| Konservierungsmittel, Farbstoffe, | q.s. |
| Parfüm | |
| Wasser | ad 100,00 |

### Beispiel 8

| Sonnenschutzemulsion | |
|---|---|
| | Gew.-% |
| Cyclomethicon | 2,00 |
| Cetylstearylalkohol + PEG 40-hydriertes Rizinusöl + Natrium | 2,50 |
| Cetylstearylsulfat | |
| Glyceryllanolat | 1,00 |
| Caprylsäure-/Caprinsäure Triglycerid | 0,10 |
| Laurylmethicon Copolyol | 2,00 |
| Octylstearat | 3,00 |
| Rizinusöl | 4,00 |
| Glycerin | 3,00 |
| Acrylamid/Natriumacrylat Copolymer | 0,30 |
| Hydroxypropylmethylcellulose | 0,30 |
| Octylmethoxycinnamat | 5,00 |
| Butyl-methoxy-dibenzoylmethan | 0,50 |
| H-VVRP-NH₂ | 0,001 |
| α-Tocopherylacetat | 1,00 |
| Na₃HEDTA | 1,50 |
| Konservierungsmittel, Farbstoffe, | q.s. |
| Parfüm | |
| Wasser | ad 100,00 |

### Beispiel 9

| Sonnenschutzemulsion | |
|---|---|
| | Gew.-% |
| Cyclomethicone | 2,00 |
| Cetylstearylalkohol +PEG 40-hydriertes Rizinusöl +Natrium | 2,50 |
| Cetylstearylsulfat | |
| Glyceryllanolat | 1,00 |
| Caprylsäure-/Caprinsäure Triglycerid | 0,10 |
| Laurylmethicon Copolyol | 2,00 |
| Octylstearat | 3,00 |
| Rizinusöl | 4,00 |
| Glycerin | 3,00 |
| Acrylamid/Natriumacrylat Copolymer | 0,30 |
| Hydroxypropylmethylcellulose | 0,30 |
| Octylmethoxycinnamat | 5,00 |
| Butyl-methoxy-dibenzoylmethan | 0,75 |
| Acetyl-VVRP-NH₂ | 0,001 |
| Na₃HEDTA | 1,50 |
| Konservierungsmittel, Farbstoffe, | q.s. |
| Parfüm | |
| Wasser | ad 100,00 |

### Beispiel 10

| Massagecrème | |
|---|---|
| | Gew.-% |
| Stearylalkohol | 2,00 |
| Petrolatum | 4,00 |
| Dimethicon | 2,00 |
| Isopropylpalmitat | 6,00 |
| Cetylstearylalkohol | 4,00 |
| PEG 40 Hydriertes Rizinusöl | 2,00 |
| α-Tocopherol | 0,50 |
| H-VVRP-NH₂ | 0,005 |
| Glycerin | 3,00 |
| Konservierungsmittel, Farbstoffe, | q.s. |
| Parfüm | |
| Wasser | ad 100,00 |

### Beispiel 11

| Haarwasser | |
|---|---|
| | Gew.-% |
| Ethanol | 40,00 |
| Diisopropyladipat | 0,10 |
| PEG-40 hydriertes Rizinusöl | 0,20 |
| H-VVRP-NH₂ | 0,001 |
| α-Tocopherylacetat | 0,10 |
| Konservierungsmittel, Farbstoffe, | q.s. |
| Parfüm | |
| Wasser | ad 100,00 |

### Beispiel 12

| Sprayformulierung | |
|---|---|
| | Gew.-% |
| α-Tocopherol | 0,10 |
| Acetyl-VVRP-NH₂ | 0,001 |
| Ethanol | 28,20 |
| Konservierungsmittel, Farbstoffe, | q.s. |
| Parfüm | |
| Propan/Butan 25/75 | ad 100,00 |

## Patentansprüche

1. Verwendung von einem oder mehreren monomeren oder homo- oder heterodimeren oder homo- oder heterotrimeren oder homo- oder heterotetrameren Oligopeptiden,
(1) wobei diesen Oligopeptiden als Monomeres eine Struktur Val-Val-Arg-Pro(bzw. VVRP) zugrundeliegt,
(2) wobei in der Sequenz Val-Val-Arg-Pro jeweils ein Valin unter Beibehaltung der Restsequenz durch eine Aminosäure aus der Gruppe Glycin, Alanin, Leucin, Isoleucin, Serin, Threonin oder Methionin, vorzugsweise Alanin, ersetzt werden kann,
(3) oder wobei in den monomeren oder homo- oder heterodimeren oder homo- oder heterotrimeren oder homo- oder heterotetrameren Oligopeptide Strukturen wie vorgenannt vorliegen mit dem Unterschiede, daß der C-Terminus und/oder der N-Terminus um bis zu 5 Aminosäuren ergänzt ist, gemäß der Struktur 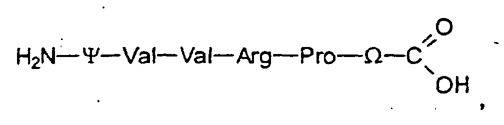 wobei Ψ und Ω unabhängig voneinander Aminosäuresequenzen von 0 bis zu 5 Aminosäuren darstellen können,
(4) oder wobei die monomeren oder homo-/hetero-di-, -tri-, oder tetrameren Oligopeptide oder Proteine am N-Terminus acyliert sind, gemäß der Struktur 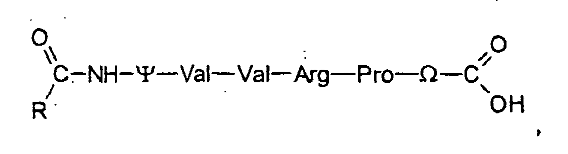 wobei Ψ und Ω unabhängig voneinander Aminosäuresequenzen von 0 bis zu 5 Aminosäuren darstellen können und R einen verzweigten oder unverzweigten, gesättigten oder ungesättigten Kohlenwasserstoffrest darstellt,
(5) oder wobei die monomeren oder homo-/hetero-di-, -tri-, oder tetrameren Oligopeptide oder Proteine am C-Terminus amidiert sind, gemäß der Struktur 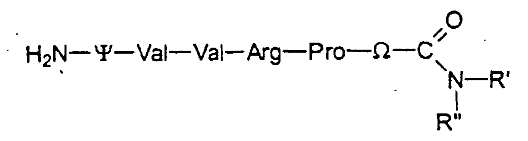 wobei Ψ und Ω unabhängig voneinander Aminosäuresequenzen von 0 bis zu 5 Aminosäuren darstellen können, und wobei R' und R" unabhängig voneinander gewählt werden können aus der Gruppe bestehend aus Wasserstoff und der verzweigten oder unverzweigten, gesättigten oder ungesättigten Kohlenwasserstoffreste,
(6) oder wobei die monomeren oder homo-/hetero-di-, -tri-, oder tetrameren Oligopeptide oder Proteine am C-Terminus amidiert und am N-Terminus acyliert sind, gemäß der Struktur 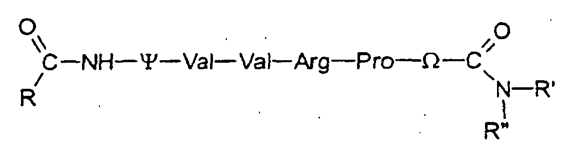 wobei Ψ und Ω unabhängig voneinander Aminosäuresequenzen von 0 bis zu 5 Aminosäuren darstellen können, R einen Alkylrest mit 1 bis 30 Kohlenstoffatomen darstellt, und wobei R' und R" unabhängig voneinander gewählt werden können aus der Gruppe bestehend aus Wasserstoff und der verzweigten oder unverzweigten, gesättigten oder ungesättigten Kohlenwasserstoffreste,
(7) oder wobei in monomeren oder homo-/hetero-di-, -tri-, oder tetrameren Oligopeptiden oder Proteinen Aminosäuresequenzen als einfach vorliegende oder sich mehrfach wiederholende Strukturmotive auftreten, gemäß den Strukturen 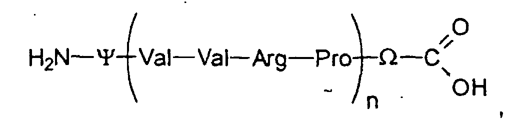 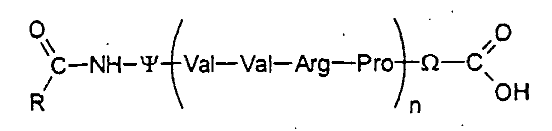 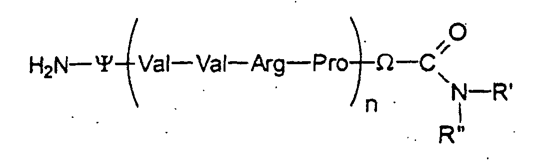 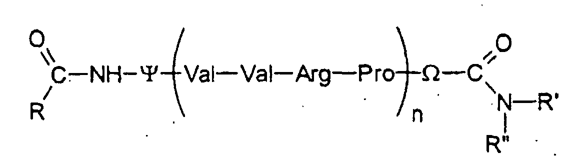
wobei Ψ eine Einfachbindung oder eine Aminosäurensequenz bis zu 5 Aminosäuren darstellen kann, Ω eine Aminosäurensequenz bis zu 15 Aminosäuren darstellen kann, n eine Zahl von 1 bis 25 darstellt, R einen verzweigten oder unverzweigten, gesättigten oder ungesättigten Kohlenwasserstoffrest, und wobei R' und R" unabhängig voneinander gewählt werden können aus der Gruppe bestehend aus Wasserstoff und der verzweigten oder unverzweigten, gesättigten oder ungesättigten Kohlenwasserstoffreste, zur Herstellung kosmetischer oder dermatologischer Zubereitungen gegen unerwünschte Pigmentierung der Haut.

2. Verwendung nach Anspruch 1, **dadurch gekennzeichnet, daß** das oder die Oligopeptide gewählt werden aus der Gruppe Val-Val-Arg-Pro, Val-Val-Arg-Pro-Pro sowie Val-Val-Arg-Pro-Pro-Pro.

3. Verwendung nach Anspruch 1, **dadurch gekennzeichnet, daß** das oder die Oligopeptide gewählt wird aus der Gruppe der mit unverzweigten Alkanoylgruppen am N-Terminus acylierten Oligopeptide Val-Val-Arg-Pro, Val-Val-Arg-Pro-Pro sowie Val-Val-Arg-Pro-Pro-Pro.

4. Verwendung nach Anspruch 1, **dadurch gekennzeichnet, daß** das oder die Oligopeptide gewählt wird aus der Gruppe 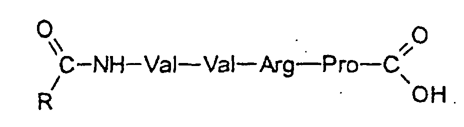 und 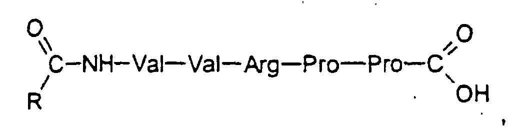 wobei R den Stearoyl oder den Palmitoylrest darstellt.

5. Verwendung nach Anspruch 1, **dadurch gekennzeichnet, daß** das oder die Oligopeptide in kosmetischen oder topischen dermatologischen Zubereitungen in Konzentrationen von 0,000001 - 10 Gew.-%, bevorzugt 0,0001 - 1 Gew.-%, insbesondere 0,0001 - 0,1 Gew.-% bezogen auf das Gesamtgewicht der Zubereitungen, vorliegt.

## Claims

1. Use of one or more monomeric or homo- or heterodimeric or homo- or heterotrimeric or homo- or heterotetrameric oligopeptides,
(1) where these oligopeptides are based on a structure Val-Val-Arg-Pro (or VVRP) as monomer,
(2) where, in the sequence Val-Val-Arg-Pro, in each case one valine may be replaced by an amino acid selected from the group consisting of glycine, alanine, leucine, isoleucine, serine, threonine and methionine, preferably alanine, while the remainder of the sequence is retained,
(3) or where structures as mentioned above are present in the monomeric or homo- or heterodimeric or homo- or heterotrimeric or homo- or heterotetrameric oligopeptides, with the difference that the C-terminus and/or the N-terminus is complemented by up to 5 amino acids, as shown by the structure 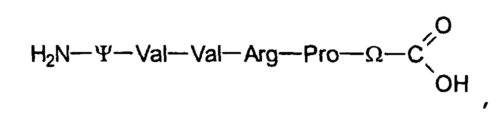 where Ψ and Ω independently of one another may represent amino acid sequences of 0 up to 5 amino acids,
(4) or where the monomeric or homo-/hetero-di-, -tri-, or -tetrameric oligopeptides or proteins are acylated on the N-terminus, as shown by the structure 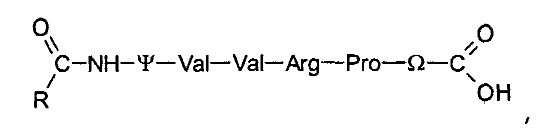 where Ψ and Ω independently of one another may represent amino acid sequences of 0 up to 5 amino acids and R represents a branched or unbranched, saturated or unsaturated hydrocarbon radical,
(5) or where the monomeric or homo-/hetero-di-, -tri-, or -tetrameric oligopeptides or proteins are amidated on the C-terminus, as shown by the structure 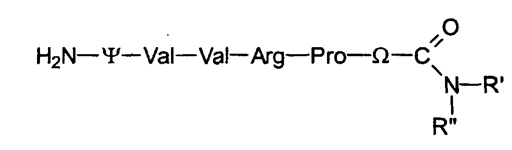 where Ψ and Ω independently of one another may represent amino acid sequences of 0 up to 5 amino acids, and where R' and R" independently of one another may be selected from the group consisting of hydrogen and the branched or unbranched saturated or unsaturated hydrocarbon radicals,
(6) or where the monomeric or homo-/hetero-di-, -tri-, or -tetrameric oligopeptides or proteins are amidated on the C-terminus and acylated on the N-terminus, as shown by the structure 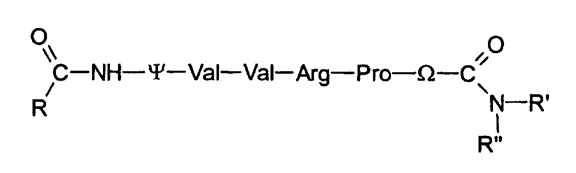 where Ψ and Ω independently of one another may represent amino acid sequences of 0 up to 5 amino acids, R represents an alkyl radical having 1 to 30 carbon atoms, and where R' and R" independently of one another may be selected from the group consisting of hydrogen and the branched or unbranched saturated or unsaturated hydrocarbon radicals,
(7) or where amino acid sequences occur in monomeric or homo-/hetero-di-, -tri- or -tetrameric oligopeptides or proteins as single or repetitive structural motifs, as shown by the structures 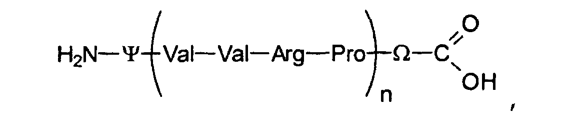 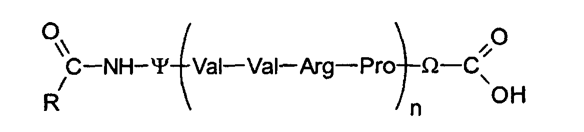 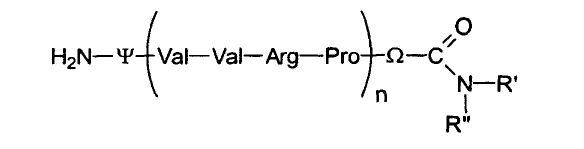 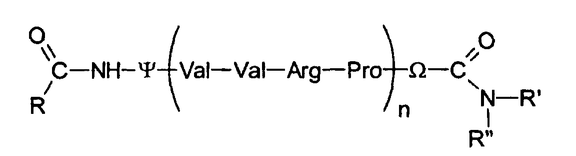
where Ψ may represent a single bond or an amino acid sequence of up to 5 amino acids, Ω may represent an amino acid sequence of up to 15 amino acids, n represents a number from 1 to 25, R represents a branched or unbranched saturated or unsaturated hydrocarbon radical, and where R' and R" independently of one another may be selected from the group consisting of hydrogen and the branched or unbranched saturated or unsaturated hydrocarbon radicals, for the production of cosmetic or dermatological perparations against undesired skin pigmentation.

2. Use according to Claim 1, **characterized in that** the oligopeptide(s) is/are selected from the group consisting of Val-Val-Arg-Pro, Val-Val-Arg-Pro-Pro and Val-Val-Arg-Pro-Pro-Pro.

3. Use according to Claim 1, **characterized in that** the oligopeptide(s) is/are selected from the group consisting of the oligopeptides Val-Val-Arg-Pro, Val-Val-Arg-Pro-Pro and Val-Val-Arg-Pro-Pro-Pro whose N-terminus is acylated by unbranched alkanoyl groups.

4. Use according to Claim 1, **characterized in that** the oligopeptide(s) is/are selected from the group consisting of 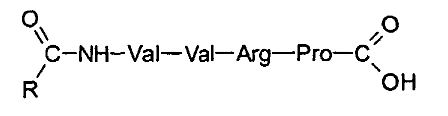 and 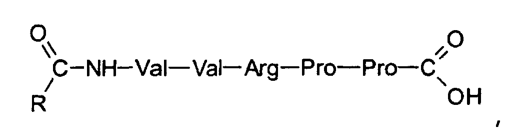 R representing the stearoyl or the palmitoyl radical.

5. Use according to Claim 1, **characterized in that** the oligopeptide(s) is/are present in cosmetic or topical dermatological preparations in concentrations of 0.000001 - 10% by weight, preferably 0.0001 - 1% by weight, in particular 0.0001 - 0.1% by weight, based on the total weight of the preparations.

## Revendications

1. Utilisation d'un ou de plusieurs oligopeptides monomères ou homodimères ou hétérodimères ou homotrimères ou hétérotrimères ou homotétramères ou hétérotétramères,
(1) ces oligopeptides ayant comme monomère une structure de base Val-Val-Arg-Pro (ou VVRP),
(2) une valine de la séquence Val-Val-Arg-Pro pouvant être remplacée à chaque fois par un acide aminé du groupe glycine, alanine, leucine, isoleucine, sérine, thréonine ou méthionine, de préférence alanine, en maintenant le reste de la séquence,
(3) ou des structures telles que susmentionnées se trouvant dans les oligopeptides monomères ou homodimères ou hétérodimères ou homotrimères ou hétérotrimères ou homotétramères ou hétérotétramères avec comme différence que la terminaison C et/ou la terminaison N est complétée avec jusqu'à 5 acides aminés selon la structure 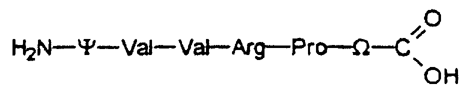 Ψ et Ω pouvant représenter indépendamment l'un de l'autre des séquences d'acides aminés de 0 à 5 acides aminés,
(4) ou les oligopeptides monomères ou homodimères ou hétérodimères ou homotrimères ou hétérotrimères ou homotétramères ou hétérotétramères ou les protéines étant acylés au niveau de la terminaison N, selon la structure 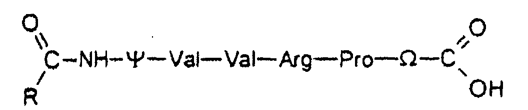 Ψ et Ω pouvant représenter indépendamment l'un de l'autre des séquences d'acides aminés de 0 à 5 acides aminés et R représentant un radical hydrocarboné ramifié ou non ramifié, saturé ou insaturé,
(5) ou les oligopeptides monomères ou homodimères ou hétérodimères ou homotrimères ou hétérotrimères ou homotétramères ou hétérotétramères ou les protéines étant amidés au niveau de la terminaison C, selon la structure 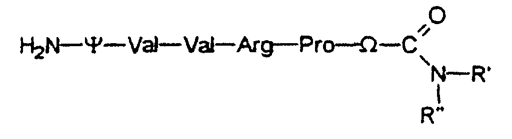 Ψ et Ω pouvant représenter indépendamment l'un de l'autre des séquences d'acides aminés de 0 à 5 acides aminés et R' et R" pouvant être choisis indépendamment l'un de l'autre dans le groupe constitué par l'hydrogène et les radicaux hydrocarbonés ramifiés ou non ramifiés, saturés ou insaturés,
(6) ou les oligopeptides monomères ou homodimères ou hétérodimères ou homotrimères ou hétérotrimères ou homotétramères ou hétérotétramères ou les protéines étant amidés au niveau de la terminaison C et acylés au niveau de la terminaison N, selon la structure 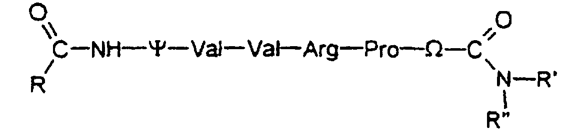 Ψ et Ω pouvant représenter indépendamment l'un de l'autre des séquences d'acides aminés de 0 à 5 acides aminés, R représentant un radical alkyle comprenant 1 à 30 atomes de carbone et R' et R" pouvant être choisis indépendamment l'un de l'autre dans le groupe constitué par l'hydrogène et les radicaux hydrocarbonés ramifiés ou non ramifiés, saturés ou insaturés,
(7) ou dans les oligopeptides monomères ou homodimères ou hétérodimères ou homotrimères ou hétérotrimères ou homotétramères ou hétérotétramères ou les protéines des séquences d'acides aminés se trouvant sous forme de motifs de structure uniques ou multiples répétés, selon les structures 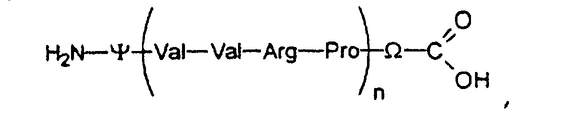 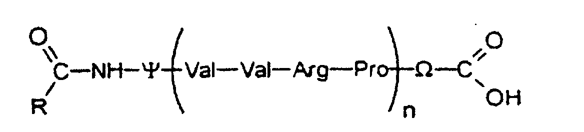 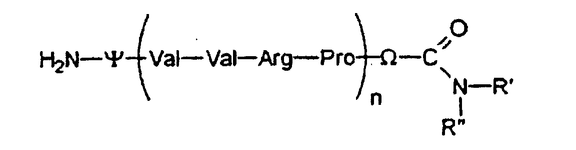 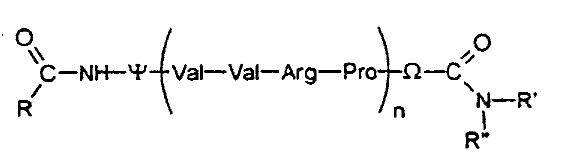 Ψ pouvant représenter une liaison simple ou une séquence d'acides aminés jusqu'à 5 acides aminés, Ω pouvant représenter une séquence d'acides aminés jusqu'à 15 acides aminés, n représentant un nombre de 1 à 25, R représentant un radical hydrocarboné ramifié ou non ramifié, saturé ou insaturé, et R' et R" pouvant être choisis indépendamment l'un de l'autre dans le groupe constitué par l'hydrogène et les radicaux hydrocarbonés ramifiés ou non ramifiés, saturés ou insaturés,
pour la préparation de compositions cosmétiques ou dermatologiques contre une pigmentation non souhaitée de la peau.

2. Utilisation selon la revendication 1, **caractérisée en ce que** le ou les oligopeptides sont choisis dans le groupe Val-Val-Arg-Pro, Val-Val-Arg-Pro-Pro ainsi que Val-Val-Arg-Pro-Pro-Pro.

3. Utilisation selon la revendication 1, **caractérisée en ce que** le ou les oligopeptides sont choisis dans le groupe des oligopeptides Val-Val-Arg-Pro, Val-Val-Arg-Pro-Pro ainsi que Val-Val-Arg-Pro-Pro-Pro acylés en terminaison N avec des groupes alcanoyle non ramifiés.

4. Utilisation selon la revendication 1, **caractérisée en ce que** le ou les oligopeptides sont choisis dans le groupe 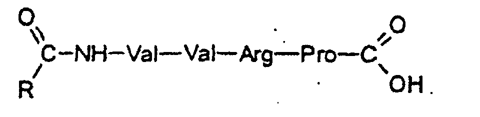 et 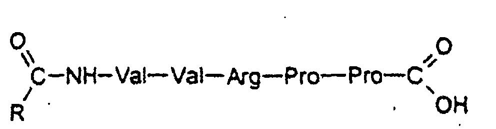 R représentant le radical stéaroyle ou palmitoyle.

5. Utilisation selon la revendication 1, **caractérisée en ce que** le ou les oligopeptides se trouvent dans les compositions dermatologiques cosmétiques ou topiques en des concentrations de 0,000001 à 10% en poids, de préférence de 0,0001 à 1% en poids, en particulier de 0,0001 à 0,1% en poids par rapport au poids total des compositions.
